(19) Europäisches Patentamt
European Patent Office
Office européen des brevets

(11) Veröffentlichungsnummer: **0 007 482**
**B1**

(12) **EUROPÄISCHE PATENTSCHRIFT**

(45) Veröffentlichungstag der Patentschrift: **16.09.81**

(21) Anmeldenummer: **79102264.3**

(22) Anmeldetag: **04.07.79**

(51) Int. Cl.³: **C 07 C 93/14,** C 07 C 103/38, C 07 C 149/42, C 07 D 295/08, A 01 N 33/22, A 01 N 43/34, A 01 N 43/72

(54) **2-Chlor-6-nitroaniline, Verfahren zu ihrer Herstellung, ihre Verwendung, herbizide Mittel.**

(30) Priorität: **15.07.78 DE 2831262**

(43) Veröffentlichungstag der Anmeldung: **06.02.80 Patentblatt 80/3**

(45) Bekanntmachung des Hinweises auf die Patenterteilung: **16.09.81 Patentblatt 81/37**

(84) Benannte Vertragsstaaten: **BE CH DE FR GB IT LU NL SE**

(56) Entgegenhaltungen:
**DD-A-105 204**
**DE-A-2 041 324**
**DE-A1-2 753 235**
**FR-A-1 499 717**
**US-A-4 039 588**
**US-A-4 046 798**
**US-A-4 105 797**

(73) Patentinhaber: **CELAMERCK GmbH & Co. KG, Binger Strasse 173, D-6507 Ingelheim am Rhein (DE)**

(72) Erfinder: **Buck, Wolfgang, Dr., In der Dörrwies 37, D-6507 Ingelheim (DE)**
Erfinder: **Sehring, Richard, Dr., Frankenstrasse 13, D-6507 Ingelheim (DE)**
Erfinder: **Linden, Gerbert, Dr., Turnierstrasse 44, D-6507 Ingelheim (DE)**
Erfinder: **Lust, Sigmund, Dr., Klappacher Strasse 2f, D-6100 Darmstadt (DE)**

**EP 0 007 482 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 2-Chlor-6-nitroaniline, Verfahren zu ihrer Herstellung, ihre Verwendung, herbizide Mittel

Die Erfindung betrifft neue 2-Chlor-6-nitroaniline, Mittel, die diese Verbindungen als Wirkstoffe enthalten, die Herstellung und die Verwendung der neuen 2-Chlor-6-nitroaniline zur Beeinflussung des Pflanzenwachstums.

Die Erfindungsgemässen 2-Chlor-6-nitroaniline entsprechen der Formel

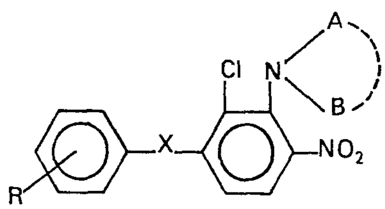

(I),

in der A für Wasserstoff, einen geradkettigen oder verzweigten Alkylrest mit 1 bis 6 C-Atomen, einen Cycloalkylrest mit 3 bis 6 C-Atomen, einen chlor- oder hydroxysubstituierten Alkylrest mit 2 bis 6 C-Atomen, Allyl oder Trifluoracetyl, B für Wasserstoff, Methyl, Äthyl, n-Propyl oder Isopropyl, A und B gemeinsam auch für einen, gegebenenfalls durch Sauerstoff, $=NH$ oder $=NCH_3$ unterbrochenen Alkylenrest mit bis zu 5 Gliedern oder die Gruppe $=CH-N(CH_3)_2$, R für Wasserstoff, Fluor, Chlor, Brom, Methyl oder Methoxy und X für Sauerstoff oder Schwefel steht.

Soweit A und B gemeinsam einen gegebenenfalls durch ein Heteroatom unterbrochenen Alkylrest darstellen, bildet dieser mit dem N-Atom der Gruppe

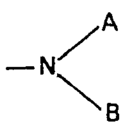 einen Heterocyclus,

etwa

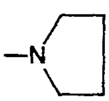, 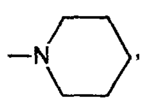, 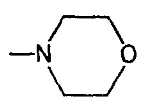,

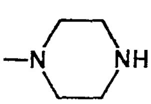, 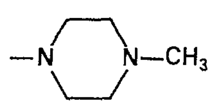

Hervorzuheben sind diejenigen Verbindungen der Formel I, bei denen

A Wasserstoff, einen $C_1$-$C_4$-Alkylrest, einen hydroxysubstituierten $C_2$-$C_4$-Alkylrest oder $COCF_3$,

B Wasserstoff, 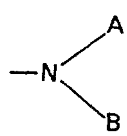 die Gruppen

$-N(CH_3)_2$ oder $-N=CH-N(CH_3)_2$,

R Wasserstoff, Fluor, Chlor, Methoxy,

X Sauerstoff darstellt.

Bevorzugt sind diejenigen Verbindungen, in denen A Wasserstoff, Methyl, Äthyl, n-Propyl, Isopropyl, n-Butyl, sec.-Butyl, tert.-Butyl, Isobutyl, Trifluoracetyl, B Wasserstoff, R Wasserstoff, Fluor oder Chlor und X Sauerstoff bedeutet.

Von den Verbindungen, in denen X Schwefel bedeutet, sind diejenigen hervorzuheben, in denen A Wasserstoff, Methyl, Äthyl oder Allyl, B Wasserstoff und R Wasserstoff oder Fluor bedeutet.

Der Rest R kann sich in 2-, 3- oder 4-Stellung befinden.

Es ist bekannt, dass einige nitrosubstituierte Diphenyläther herbizide Eigenschaften haben (vgl. K.H. Büchel, Pflanzenschutz und Schädlingsbekämpfung, Georg Thieme Verlag, Stuttgart 1977, S. 166).

Wie nun gefunden wurde, zeichnen sich nun die aminosubstituierten Verbindungen der Formel I durch hervorragende herbizide Wirkung gegen eine grosse Zahl von Unkräutern und Ungräsern aus. Zudem können die neuen Verbindungen auch selektiv in zahlreichen Nutzpflanzenkulturen angewendet werden.

Die neuen Verbindungen werden in an sich bekannter Weise hergestellt.

1.) Durch Umsetzung von Phenolen oder Thiophenolen der Formel

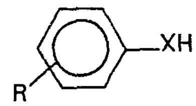

(II),

worin R und X die oben angegebene Bedeutung haben, in Gegenwart eines säurebindenden Mittels oder – bevorzugt – in Form eines entsprechenden Phenolats bzw. Thiophenolats, insbesondere des Natrium- oder Kaliumsalzes, mit einem 2,3-Dichlor-6-nitroanilin der Formel

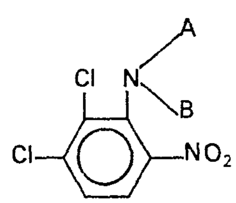

(III)

worin A und B die oben angegebene Bedeutung haben, bei einer Temperatur zwischen Raumtemperatur und etwa 120 °C, vorzugsweise zwischen 50 und 100 °C.

Als Reaktionsmedium dient ein unter den Reaktionsbedingungen inertes Lösungsmittel, insbesondere Acetonitril oder Dimethylformamid, ferner z.B. Methyläthylketon, Dimethylsulfoxid, N-Methylpyrrolidon.

Die Ausgangsstoffe der Formeln II und III werden nach üblichen Verfahren gewonnen. Für die Herstellung der Aniline III, die zum Teil neu sind, kann man 2,3,4-Trichlornitrobenzol mit 2 Mol des

Amins HNAB in einem geeigneten Lösungsmittel umsetzen. Ein Mol des Amins HNAB kann vorteilhaft durch ein tertiäres Amin ersetzt werden. Im allgemeinen kann das Anilin III ohne Reinigung für die weitere Umsetzung verwendet werden. Mitunter ist es jedoch vorteilhaft, III durch Destillation, Umkristallisieren oder Säulenchromatographie zu reinigen. Die Verwendung eines gereinigten Anilins der Formel III führt im allgemeinen auch zu einer besseren Gesamtausbeute und liefert unmittelbar ein hinreichend reines Endprodukt.

2.) Zur Herstellung solcher Verbindungen der Formel I, in denen A einen chlorsubstituierten Alkylrest bedeutet, ersetzt man mit den dazu üblichen Mitteln in den entsprechenden N-Hydroxyalkylverbindungen der Formel I die Hydroxygruppe durch Chlor.

Diese Umsetzung wird nach an sich bekannten Verfahren, gegebenenfalls in einem inerten Lösungsmittel, etwa Toluol, durchgeführt. Bei der Anwendung von Phosphorpentachlorid verläuft die Reaktion unter spontaner Erwärmung, bei der Anwendung von Thionylchlorid ist Erwärmen vorteilhaft.

Die Ausgangsstoffe können nach üblichen Verfahren erhalten werden, z.B. nach dem obigen Verfahren zur Herstellung von Verbindungen der Formel I.

3.) Solche Verbindungen der Formel I, in denen A Trifluoracetyl bzw. A und B gemeinsam die Gruppe $=CH-N(CH_3)_2$ bedeuten, lassen sich aus entsprechenden Verbindungen, in denen A bzw. A und B für Wasserstoff stehen, durch Umsetzung mit Trifluoracetanhydrid bzw. mit Dimethylformamid/Phosphoroxychlorid oder einem Dimethylformamiddialkylacetal erhalten.

Für die Einführung der Trifluoracetylgruppe bringt man die Ausgangsverbindung zweckmässig in einem inerten Lösungsmittel, etwa Toluol, bei Raumtemperatur oder unter Erwärmen mit dem Trifluoracetanhydrid zur Reaktion.

Für die Herstellung des Amidins wird das Reaktionsgemisch stärker erwärmt (auf etwa 80–150 °C). Als Lösungsmittel kann Dimethylformamid, aber beispielsweise auch Dimethylsulfoxid dienen.

Die Ausgangsstoffe können nach Verfahren 1.) gewonnen werden.

Die neuen Verbindungen der Formel I stellen auch wertvolle Zwischenprodukte für Pflanzenschutzmittel und Arzneistoffe dar, weil sie aufgrund der Amino- und der Nitrogruppen vielfältig abgewandelt werden können.

Für die Anwendung als Herbizide werden die Verbindungen der Formel I in an sich bekannter Weise mit üblichen Hilfs- und/oder Trägerstoffen zu gebräuchlichen Formulierungen verarbeitet, z.B. zu Emulsionskonzentraten oder Suspensionspulvern, bei denen der Wirkstoffgehalt zwischen etwa 10 und 95 Gewichtsprozent liegt und die für die Ausbringung mit Wasser auf die gewünschte Wirkstoffkonzentration eingestellt werden. Jedoch können auch Präparate hergestellt werden, die unverdünnt angewendet werden, beispielsweise Stäube oder Granulate. Hier liegt der Wirkstoffgehalt zwischen 0,2 und 20 Gewichtsprozent, vorzugsweise zwischen 0,5 und 3 Gewichtsprozent.

Formulierungsbeispiele:

1. Suspensionspulver

Zusammensetzung:

25 Gew.-% 2-Chlor-3-phenoxy-6-nitroanilin
55 Gew.-% Kaolin
10 Gew.-% kolloidale Kieselsäure
9 Gew.-% Calciumligninsulfonat
1 Gew.-% Natriumtetrapropylenbenzolsulfonat

2. Emulsionskonzentrat

20 Gew.-% einer Verbindung der Formel I
70 Gew.-% flüssiges Lösungsmittelgemisch aus hochsiedenden aromatischen Kohlenwasserstoffen (Shellsol A)
6,5 Gew.-% Tensiofix AS (Emulgator)
3,5 Gew.-% Tensiofix DS (Emulgator)

Aus den Konzentraten nach Beispiel 1 und 2 werden durch Vermischen mit Wasser Spritzbrühen hergestellt, die im allgemeinen etwa 0,05 bis 0,5 Gewichtsprozent Wirkstoff enthalten.

3. Stäubemittel

1 Gew.-% einer Verbindung der Formel I
98 Gew.-% Talkum
1 Gew.-% Methylcellulose

Es ist auch möglich und in einigen Fällen vorteilhaft, die erfindungsgemässen Wirkstoffe zusammen mit anderen Herbiziden einzusetzen, z.B. mit Triazin-Herbiziden wie Simazin oder Atrazin in Mais, mit Harnstoff-Herbiziden wie z.B. Linuron oder Monolinuron in Kartoffeln oder Getreide, mit Dinitroanilin-Herbiziden wie Dinitramin oder Trifluoralin in Getreide, mit Diphenyläthern, z.B. Nitrophen in Reis, mit Carbonsäureamiden wie Alachlor in Zwiebeln.

Die erfindungsgemässen Verbindungen können im Vorauflaufverfahren und im Nachauflaufverfahren angewendet werden. Beispielsweise können im Freiland mit einer Aufwandmenge von 1 kg/ha 2-Chlor-3-Phenoxy-6-nitroanilin bei Vorauflaufanwendung Senf, Amaranth, Gänsefuss, Kamille, Hühnerhirse und auch Ackerfuchsschwanz bekämpft werden. Dabei beträgt die Verträglichkeit in Kartoffeln und Mais über 3 kg/ha, in Weizen und Erbsen über 2,5 kg/ha. Die Anwendung der genannten Verbindungen nach dem Auflaufen ermöglicht es, mit 1 kg/ha z.B. Labkraut, Gänsefuss, Ackerfuchsschwanz, mit geringfügig höherer Dosis auch Senf, Amaranth und Kamille zu bekämpfen. Ausser in den genannten Kulturen können die erfindungsgemässen Verbindungen z.B. in Gerste, Rüben und Reis eingesetzt werden.

Aus der DD-A-105 204 ist eine allgemeine Formel bekannt, unter die, bei entsprechender Konkretisierung der Substituentenbedeutungen, auch erfindungsgemässe Verbindungen fallen. In der genannten Patentschrift sind jedoch keine Verbindungen beschrieben, welche die für die erfindungsgemässen Verbindungen typische Substituentenfolge Nitrogruppe, Aminogruppe, Chlor, Phenoxy- bzw. Phenylthiogruppe aufweisen. Auch sind die Verbindungen nur als «Pflanzenschutzmittel» bezeichnet; ein Hinweis auf die Verwendbarkeit der Verbindungen zur Beeinflussung des Pflanzenwachstums findet sich nicht.

Die in US-A-4 046 798 als herbizid wirksam beschriebenen Verbindungen unterscheiden sich konstitutionell deutlich von den erfindungsgemässen Verbindungen. Zum einen fehlt die für diese typische Chlorsubstitution in 2-Stellung zur Aminogruppe, zum anderen weisen alle Verbindungen der US-Patentschrift zwingend die 4 – $CF_3$ – Gruppe im Phenoxyteil auf. Überdies enthält die Phenoxygruppe zusätzlich stets noch ein Chloratom in 2-Stellung.

Den erfindungsgemässen Verbindungen am ähnlichsten sind die in der US-A-4 039 588 beschriebenen Verbindungen, obgleich auch diese in der nitrosubstituierten Phenylgruppe nicht das 2-Chloratom aufweisen, im Phenoxyteil jedoch mindestens zwei Chloratome enthalten. Aus dieser US-Patentschrift wurden zwei Verbindungen [2-Nitro-5-(2,4-dichlorphenoxy)-N,N-dimethylanilin (I; Tabelle I, Example 8) und 2-Nitro-5-(2,4-dichlorphenoxy)-N-isopropylanilin (II; Tabelle I, Example 15)] zum Vergleich herangezogen.

In der nachstehenden Tabelle sind Ergebnisse von Gewächshausversuchen für eine repräsentative Auswahl erfindungsgemässer Verbindungen sowie die Vergleichsverbindungen I und II bei wichtigen Unkräutern und Ungräsern aufgeführt.

Angegeben ist die Aufwandmenge in kg/ha.

A: Sinapis alba (Weisser Senf, white mustard)
B: Galium aparine (Klettenlabkraut, catchweed bedstraw)
C: Alopecurus myosuroides (Ackerfuchsschwanz, blackgrass)
D: Echinochloa crus-galli (Hühnerhirse, barnyardgrass).

VA bedeutet Anwendung im Vorauflauf-, NA im Nachauflaufverfahren.

| Verbindung | Anwendungszeit | A | B | C | D |
|---|---|---|---|---|---|
| Tab. Nr. 10 | NA | <1 | <1 | 1 | 4 |
| Tab. Nr. 12 | VA | <1 | <1 | 4 | 4 |
| Tab. Nr. 13 | NA | <1 | 4 | 4 | 4 |
| Beisp. 4 | NA | <1 | 4 | 4 | 4 |
| Beisp. 7 | NA | <1 | <1 | 4 | 4 |
| Tab. Nr. 22 | VA | 1 | 1 | <1 | 4 |
| Tab. Nr. 15 | VA | <1 | 1,25 | <1 | 1,25 |
| | NA | <1 | <1 | <1 | <1 |
| Tab. Nr. 28 | NA | <1 | 1,25 | <1 | <1 |
| Tab. Nr. 40 | VA | 1,25 | 1,25 | 1,25 | 1,25 |
| Tab. Nr. 19 | NA | <1 | 1,25 | 1,25 | 1,25 |
| Tab. Nr. 37 | NA | 1,25 | 1,25 | 1,25 | 1,25 |
| Tab. Nr. 62 | VA | 1 | 4 | 1 | 4 |
| Beisp. 1 | VA | <1 | 1 | 1 | <1 |
| I (Vergleich) | VA | >4 | >4 | >4 | 1 |
| | NA | >4 | 1 | >4 | >4 |
| II (Vergleich) | VA | >4 | >4 | >4 | >4 |

Wie die Zahlenwerte zeigen, erweisen sich die erfindungsgemässen Verbindungen grösstenteils als deutlich besser wirksam.

Herstellung von Ausgangsstoffen der Formel III
Beispiel:
2,3-Dichlor-6-nitro-N-isobutylanilin

75,5 g 2,3,4-Trichlornitrobenzol und 48 g Isobutylamin werden in 200 ml Toluol gelöst und 3 Tage bei Raumtemperatur gehalten. Dann wird der Ansatz mit weiteren 5 g Isobutylamin versetzt und 4 Stunden auf 60°C erwärmt. Die Toluollösung wird mit Wasser ausgeschüttelt, getrocknet und eingeengt. Man erhält 86 g (98% d.Th.) der Titelverbindung als bräunliches Öl.

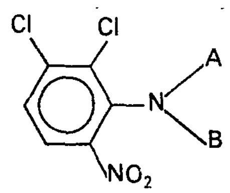

(III)

| A | B | Fp. oder $n_D^{20}$ | Lösungs-mittel | Reaktions-Temperatur | Zeit |
|---|---|---|---|---|---|
| H | H | 166 °C | DMSO | 50 °C | 24 Std. |
| $CH_3$ | H | 60 °C | DMSO | 10 °C | 3 Std. |
| $C_2H_5$ | H | 51 °C | DMSO | RT | 4 Std. |
| $C_2H_5$ | $C_2H_5$ | 1,564 | DMSO | RT | 5 Std. |
| $CH_3$ | $CH_3$ | 1,590 | DMF | RT | 5 Std. |
| $C_3H_7$ | H | 1,627 | DMSO | RT | 3 Std. |
| $i\text{-}C_3H_7$ | H | 1,615 | DMSO | RT | 26 Std. |
| $n\text{-}C_4H_9$ | H | 1,613 | DMSO | RT | 2 Tage |
| $n\text{-}C_3H_7$ | $n\text{-}C_3H_7$ | 1,557 | ohne | 80 °C | 9 Std. |
| $(CH_3)_2CHCH_2$ | H | 1,605 | Toluol | RT | 3 Tage |
| $H_5C_2CHCH_3$ | H | 1,603 | Toluol | 80 °C | 8 Std. |
| $HOCH_2CH_2$ | H | 94-5 °C | DMSO | RT | 16 Std. |
| $(CH_2)_5$ | | 73-4 °C | Dioxan | RT | 3 Tage |
| $(CH_2)_4$ | | 1,600 | Toluol | RT | 2 Tage |
| $-(CH_2)_2-N-(CH_2)_2-$<br>$CH_3$ | | 93-4 °C | Dioxan | 100 °C | 10 Std. |
| $-(CH_2)_2-O-(CH_2)_2-$ | | 107-8 °C | Dioxan | 100 °C | 10 Std. |
| $n\text{-}C_6H_{13}$ | H | 1,591 | Toluol | RT | 2 Tage |
| $n\text{-}C_5H_{11}$ | H | 1,593 | Toluol | RT | 2 Tage |
| 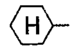 | H | 1,615 | Toluol | RT | 3 Tage |
| $CH_2{=}CH\text{-}CH_2\text{-}$ | H | 1,636 | Toluol | RT | 3 Tage |

DMSO: Dimethylsulfoxid RT: Raumtemperatur
DMF: Dimethylformamid Std.: Stunden

Beispiel 1

a) 2,3-Dichlor-6-nitroanilin

453 g 2,3,4-Trichlornitrobenzol (2 Mol) werden in 2 Liter Dimethylsulfoxid gelöst und in einen 5 Ltr. fassenden Autoklav gefüllt. 200 ml flüssiges Ammoniak (8 Mol) werden zugegeben. Nach dem Verschliessen des Autoklavs wird der Ansatz unter Rühren oder Schütteln auf 50 °C geheizt und 24 Stunden bei dieser Temperatur gehalten.

Nach Abkühlen und Belüften des Autoklavs wird die erhaltene Lösung mit ausgefallenem $NH_4Cl$ auf 8 Ltr. Wasser gegossen. Das ausgefallene Produkt wird abgesaugt, mit Wasser nachgewaschen und bei 50 °C über Nacht getrocknet. Man erhält 400 g (96,5% d.Th.) eines gelben, kristallinen Produkts, das bei 161–164 °C schmilzt. Das Produkt ist für die weiteren Umsetzungen genügend rein. Es kann jedoch auch durch Umkristallisieren, z.B. aus Methylisobutylketon, gereinigt werden.

Ausbeute dann 77% d.Th., Fp. 166–167 °C.

b) 2-Chlor-3-phenoxy-6-nitroanilin

b 1) Aus rohem 2,3-Dichlor-6-nitroanilin

Eine Lösung von 103,5 g 2,3-Dichlor-6-nitroanilin (0,5 Mol) und 63,8 g Natriumphenolat (0,55 Mol) in 1000 ml Acetonitril wird 5 Stunden unter Rückfluss erhitzt und anschliessend eingeengt. Der Rückstand wird in 750 ml Chloroform gelöst und die Lösung zweimal mit je 200 ml Wasser ausgeschüttelt. Nach dem Trocknen der Chloroformlösung wird diese eingeengt. Der Rückstand, 130 g eines braunen Öls, wird in ca. 700 ml Isopropanol heiss gelöst und die Lösung unter Rühren abgekühlt. Die Titelverbindung fällt aus und wird abgesaugt. Man erhält 98 g gelbe Kristalle (74% d.Th.) mit dem Schmelzpunkt 83 °C.

Das NMR-Spektrum bestätigt die Struktur.

b 2) Aus gereinigtem 2,3-Dichlor-6-nitroanilin

15,5 g gereinigtes 2,3-Dichlor-6-nitroanilin werden in 75 ml Dimethylsulfoxid gelöst, mit 7,8 g Phenol und 11,5 g gemahlenem Kaliumcarbonat versetzt und 8 Stunden bei 50 °C gerührt. Anschliessend wird der Ansatz mit Eisessig angesäuert, das Produkt mit Eiswasser ausgefällt, abgesaugt und getrocknet. Man erhält 19,5 g Endprodukt (98% Ausbeute bezogen auf eingesetztes 2,3-Dichlor-6-nitroanilin). Gelbe Kristale, Fp. 80–82 °C.

Beispiel 2

2-Chlor-3-phenoxy-6-nitroanilin

Eine Lösung von 911 g (4 Mol) 2,3,4-Trichlornitrobenzol werden in 4 l Dimethylsulfoxid gelöst und in einem 10-l-Autoklav mit 400 ml flüssigem Ammoniak versetzt. Der Autoklav wird verschlossen und 24 Stunden auf 50 °C gehalten, wobei sich ein Druck von 5,3 bar einstellt. Nach Abkühlen des Ansatzes und Belüften des Druckgefässes wird der Ansatz entnommen und in einem 15-l-Gefäss mit 400 g 40%iger (Gewichtsprozent) Natronlauge verrührt. Hierbei entweicht Ammoniak, das abgezogen wird. Dann gibt man 416 g Phenol (4,4 Mol) zu der Lösung, erwärmt auf 50 °C und tropft 440 g 40%ige (Gewichtsprozent) Natronlauge zu und rührt den Ansatz 7 Stunden bei 50–60 °C. Zur Vervollständigung der Reaktion werden nochmal 41 g Phenol und 44 g 40%ige (Gewichtsprozent) Natronlauge zugesetzt und weitere 3 Stunden bei 60 °C gehalten. Man lässt den Ansatz abkühlen, säuert ihn mit 100 ml Eisessig an, und fällt das Produkt mit Eiswasser unter Anreiben aus. Das gelbbraune, feste Rohprodukt wird abgesaugt, mit Wasser gewaschen und getrocknet. Das Rohprodukt (980 g, Fp. 70–78 °C) wird aus 2,5 l Isopropanol umkristallisiert.

Ausbeute: 800 g (75,5% d.Th.), gelbbrauner Feststoff. Fp. 81,5–83,5 °C.

Beispiel 3:

2-Chlor-3-phenylthio-6-nitro-N-methylanilin

Zu einer Lösung von 4,4 g (0,02 Mol) 2,3-Dichlor-6-nitro-N-methylanilin, gelöst in 50 ml Acetonitril, gibt man 2,9 g Natriumthiophenolat. Der Ansatz wird ½ Stunde unter Rückfluss erhitzt, anschliessend eingeengt und der Rückstand mit Chloroform und Wasser aufgenommen. Die Chloroformschicht wird abgetrennt, getrocknet und eingeengt. Man erhält 6 g braunes Öl, welches mit Isopropanol kristallisiert.

4 g gelbe Kristalle, Fp. 79 °C (67,5% d.Th.).

| Analyse: | C | H | N | Cl | S |
|---|---|---|---|---|---|
| ber.: | 52,7% | 3,72% | 9,47% | 12,05% | 10,9% |
| gef.: | 52,73% | 3,75% | 9,55% | 12,00% | 10,82% |

Beispiel 4

2-Chlor-3-(3-chlorphenoxy)-6-nitro-trifluoracetanilid

90 g 2-Chlor-3-(3-chlorphenoxy)-6-nitroanilin werden in 500 ml Toluol teilweise gelöst. Unter Rühren werden 70 g Trifluoracetanhydrid während 15 Min. zugetropft. Der Ansatz wird über Nacht nachgerührt. Dann wird das Lösungsmittel abdestilliert, der Rückstand wird in 300 ml Äther gelöst und das Produkt durch Zusatz von Benzin unter Reiben ausgefällt, abgesaugt und getrocknet. Man erhält 101 g hellbeige gefärbten, kristallinen Feststoff, Fp. 99–101 °C (85% d.Th.).

Beispiel 5:

1,1-Dimethyl-3-(2-chlor-3-phenoxy-6-nitrophenyl)-formamidin

7,9 g 2-Chlor-3-phenoxy-6-nitroanilin werden mit 10 ml Dimethylformamid und 10 ml Dimethylformamiddimethylacetal 8 Stunden auf 100 bis 110 °C erhitzt. Anschliessend wird der Ansatz im Vakuum bei 90 °C eingeengt. Man erhält eine rotbraune, zähe Masse, die bei ca. 0 °C erstarrt.

Die Analyse bestätigt die angegebene Formel.

| Analyse: | C | H | N | Cl |
|---|---|---|---|---|
| ber.: | 56,34% | 4,41% | 13,14% | 11,09% |
| gef.: | 56,34% | 4,39% | 13,17% | 11,09% |

Beispiel 6:

2-Chlor-3-(4-chlorphenoxy)-6-nitro-N-2-chloräthylanilin

4,5 g 2-Chlor-3-(4-chlorphenoxy)-6-nitro-N-2-hydroxyäthylanilin werden in 40 ml Toluol gelöst und mit 2,9 g $PCl_5$ versetzt, wobei Selbsterwärmung und eine Gasentwicklung eintreten. Nach 30 Minuten wird der Ansatz mit Wasser und Na-Bicarbonatlösung ausgeschüttelt und eingeengt. Man erhält 3,6 g gelbbraunes Öl (76% d.Th.).

Beispiel 7:

2-Chlor-3-phenyltio-6-nitroanilin

103,5 g 2,3-Dichlor-6-nitroanilin (0,5 Mol) und 72,5 g Natriumthiophenolat (0,55 Mol) werden in 800 ml Acetonitril gelöst und 5 Stunden unter Rückfluss gehalten. Der Ansatz wird entsprechend dem Beispiel 1 b1) aufgearbeitet. Man erhält 108 g ockerfarbenes kristallines Produkt (77% d.Th.), Fp. 144–146 °C.

Beispiel 8:

2-Chlor-3-(3-chlorphenoxy)-6-nitroanilin

103,5 g 2,3-Dichlor-6-nitroanilin (0,5 Mol) und 82,8 g Natrium-3-chlorphenolat werden in 500 ml Dimethylformamid eine Stunde auf 100 °C erhitzt. Nach Einengen der Lösung wird der Rückstand in Chloroform aufgenommen, mit Wasser gewaschen und die getrocknete Chloroformlösung eingeengt.

Der ölige Rückstand wird in ca. 700 ml Isopropanol warm gelöst. Beim Abkühlen kristallisiert die Titelverbindung. Man erhält 90 g eines ockerfarbenen kristallinen Stoffes mit dem Schmelzpunkt 101–102 °C.

Beispiel 9:

2-Chlor-3-(4-fluorphenoxy)-6-nitro-N-äthylanilin

23,5 g (0,1 Mol) N-Äthyl-2,3-dichlor-6-nitroanilin werden in 150 ml Dimethylsulfoxid in einem 250 ml Dreihalskolben gelöst. Nach Zugabe von 12,3 g (0,11 Mol) 4-Fluorphenol und 15,2 g (0,11 Mol) Kaliumcarbonat wird der Ansatz 5 Stunden bei 80 °C gerührt. Das Produkt wird durch langsames Zugeben von Eiswasser aus der abgekühlten Lösung ausgefällt und nach dem Absaugen aus Isopropanol umkristallisiert.

Man erhält 17 g ockerfarbenes, kristallines Produkt, Fp. 69–70 °C (54,7% d.Th.).

| Analyse: | C | H | N | Cl | F |
|---|---|---|---|---|---|
| ber.: | 50,99% | 2,85% | 9,92% | 12,55% | 6,72% |
| gef.: | 51,17% | 3,01% | 9,92% | 12,35% | 6,54% |

Entsprechend den vorstehenden Beispielen werden auch die folgenden Verbindungen erhalten:

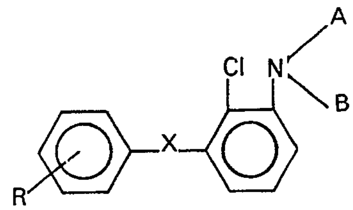

(I),

| Nr. | R | X | A | B | Fp [°C] | RF-Werte (a) | (b) |
|---|---|---|---|---|---|---|---|
| 10 | 4-Cl | O | H | H | 108 | | |
| 11 | 4-$CH_3$ | O | H | H | 97–98 | | |
| 12 | 4-$OCH_3$ | O | H | H | 113–115 | | |
| 13 | 3-$CH_3$ | O | H | H | 112–114 | | |
| 14 | 4-Cl | S | H | H | 136–138 | | |
| 15 | 4-F | O | H | H | 111–113 | | |
| 16 | 2-Cl | O | H | H | 85–86 | | |
| 17 | 4-Br | O | H | H | 118 | | |
| 18 | 3-F | O | H | H | 58–60 | | |
| 19 | 2-F | O | H | H | 104–105 | | |
| 20 | 4-Cl | O | $COCF_3$ | H | 135–137 | | |
| 21 | 4-$CH_3$ | O | $COCF_3$ | H | 155 | | |
| 22 | H | O | $CH_3$ | H | 76 | | |
| 23 | 4-Cl | O | $CH_3$ | H | 75–77 | | |
| 24 | 3-Cl | O | $CH_3$ | H | Öl | | |
| 25 | 4-$CH_3O$ | O | $CH_3$ | H | 100–101 | | |
| 26 | 4-F | O | $CH_3$ | H | 103 | | |
| 27 | H | O | $C_2H_5$ | H | Öl | | |
| 28 | 4-F | O | $C_2H_5$ | H | 71–72 | | |
| 29 | 4-Cl | O | $C_2H_5$ | H | 72–74 | | |
| 30 | 4-$CH_3$ | O | $C_2H_5$ | H | 51–52 | | |
| 31 | H | S | $C_2H_5$ | H | Öl | 0,6 | 0,45 |
| 32 | 3-$CH_3$ | O | $C_2H_5$ | H | Öl | 0,61 | 0,445 |
| 33 | 3-Cl | O | $C_2H_5$ | H | Öl | 0,60 | 0,48 |
| 34 | 4-$CH_3O$ | O | $C_2H_5$ | H | 47–48 | | |
| 35 | 4-Br | O | $C_2H_5$ | H | 57–59 | | |
| 36 | 2-Cl | O | $C_2H_5$ | H | Öl | 0,58 | 0,045 |
| 37 | H | O | n-$C_3H_7$ | H | Öl | 0,615 | 0,47 |
| 38 | H | O | -$CH(CH_3)_2$ | H | Öl | 0,61 | 0,45 |
| 39 | H | O | $CH_3$ | $CH_3$ | 69–72 | | |
| 40 | 4-F | O | $CH_3$ | $CH_3$ | 43–44 | | |
| 41 | H | O | $C_2H_5$ | $C_2H_5$ | Öl | | |
| 42 | 4-Cl | O | n-$C_3H_7$ | H | Öl | 0,63 | 0,525 |
| 43 | 4-Cl | O | -$CH(CH_3)_2$ | H | Öl | 0,63 | 0,50 |
| 44 | H | O | n-$C_4H_9$ | H | Öl | 0,625 | 0,50 |
| 45 | 4-F | O | n-$C_3H_7$ | H | Öl | 0,61 | 0,48 |
| 46 | 4-F | O | $CH(CH_3)_2$ | H | Öl | 0,62 | 0,46 |
| 47 | 4-$CH_3$ | O | n-$C_3H_7$ | H | Öl | 0,64 | 0,47 |
| 48 | H | O | n-$C_3H_7$ | n-$C_3H_7$ | Öl | | |
| 49 | 4-F | O | n-$C_4H_9$ | H | Öl | 0,63 | 0,505 |
| 50 | 4-Cl | O | n-$C_4H_9$ | H | Öl | 0,64 | 0,55 |
| 51 | 4-$CH_3$ | O | n-$C_4H_9$ | H | Öl | 0,64 | 0,495 |
| 52 | 4-$CH_3O$ | O | n-$C_3H_7$ | H | Öl | 0,59 | 0,29 |
| 53 | 4-$CH_3O$ | O | -$CH(CH_3)_2$ | H | Öl | 0,57 | 0,28 |
| 54 | 4-$CH_3O$ | O | n-$C_4H_9$ | H | Öl | 0,555 | 0,32 |
| 55 | 4-Br | O | n-$C_3H_7$ | H | Öl | 0,59 | 0,52 |
| 56 | 4-Br | O | n-$C_4H_9$ | H | Öl | 0,61 | 0,555 |

| Nr. | R | X | A | B | Fp. [°C] | RF-Werte (a) | (b) |
|---|---|---|---|---|---|---|---|
| 57 | H | O | $CH_2CH(CH_3)_2$ | H | Öl | 0,60 | 0,51 |
| 58 | H | O | $-CHC_2H_5$<br>$CH_3$ | H | Öl | 0,61 | 0,50 |
| 59 | 4-F | O | $CH_2CH(CH_3)_2$ | H | Öl | 0,60 | 0,525 |
| 60 | 4-F | O | $CHC_2H_5$<br>$CH_3$ | H | Öl | 0,62 | 0,515 |
| 61 | 4-Cl | O | $CH_2CH(CH_3)_2$ | H | Öl | 0,63 | 0,56 |
| 62 | H | O | $C_2H_4OH$ | H | Öl | 0,445 | 0,01 |
| 63 | H | S | $n-C_3H_7$ | H | Öl | 0,62 | 0,49 |
| 64 | H | S | $-CH(CH_3)_2$ | H | 56–57 | | |
| 65 | H | S | $n-C_4H_9$ | H | Öl | 0,63 | 0,52 |
| 66 | H | S | $CH_2CH(CH_3)_2$ | H | Öl | 0,78 | 0,52 |
| 67 | 4-F | O | $-C_2H_4OH$ | H | Öl | 0,44 | 0,01 |
| 68 | $4-CH_3O$ | O | $CH_2CH(CH_3)_2$ | H | Öl | 0,58 | 0,35 |
| 69 | H | O | $-(CH_2)_4-$ | | 106–107 | | |
| 70 | H | O | $-(CH_2)_5-$ | | 98–99 | | |
| 71 | H | O | $-(CH_2)_2-N-(CH_2)_2-$<br>$CH_3$ | | 99–101 | | |
| 72 | H | O | $-(CH_2)_2-O-(CH_2)_2-$ | | 109–110 | | |
| 73 | H | S | $CH_3$ | H | 128–130 | | |
| 74 | 4-F | O | $n-C_6H_{13}$ | H | Öl | 0,63 | 0,57 |
| 75 | H | O | $n-C_5H_{11}$ | H | Öl | 0,62 | 0,54 |
| 76 | 4-Cl | O | $-(CH_2)_5-$ | | 105 | | |
| 77 | 4-F | O | $n-C_5H_{11}$ | H | Öl | 0,62 | 0,56 |
| 78 | 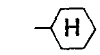 | O | H | H | Öl | 0,61 | 0,52 |
| 79 | H | S | $-CH_2-CH=CH_2$ | H | Öl | 0,72 | 0,48 |

(a) Aceton/Heptan 1:1
(b) Toluol

**Patentansprüche:**

1. 2-Chlor-6-nitroaniline der Formel

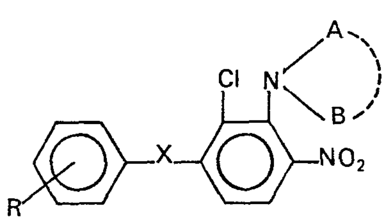

(I),

in der A für Wasserstoff, einen geradkettigen oder verzweigten Alkylrest mit 1 bis 6 C-Atomen, einen Cycloalkylrest mit 3 bis 6 C-Atomen, einen Cycloalkylrest mit 3 bis 6 C-Atomen, einen chlor- oder hydroxysubstituierten Alkylrest mit 2 bis 6 C-Atomen, Allyl oder Trifluoracetyl, B für Wasserstoff, Methyl, Äthyl, n-Propyl oder Isopropyl, A und B gemeinsam auch für einen, gegebenenfalls durch Sauerstoff, =NH oder $=NCH_3$ unterbrochenen Alkylenrest mit bis zu 5 Gliedern oder die Gruppe $=CH-N(CH_3)_2$, R für Wasserstoff, Fluor, Chlor, Brom, Methyl oder Methoxy und X für Sauerstoff oder Schwefel steht.

2. 2-Chlor-3-phenoxy-6-nitroanilin.

3. Herbizide Mittel, gekennzeichnet durch einen Gehalt an einer Verbindung nach Anspruch 1 oder 2.

4. Verwendung von Verbindungen nach Anspruch 1 oder 2 zur Bekämpfung unerwünschten Pflanzenwuchses.

5. Verwendung von Verbindungen nach Anspruch 1 oder 2 zur selektiven Unkraut- und Ungrasbekämpfung in Mais, Weizen, Gerste, Reis, Rüben und Kartoffeln.

6. Verfahren zur Herstellung von Verbindungen nach Anspruch 1 oder 2, dadurch gekennzeichnet, dass man

a) in an sich bekannter Weise ein Phenol oder Thiophenol der Formel

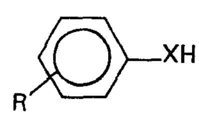

(II),

gegebenenfalls in Form eines Salzes, insbesondere eines Alkalisalzes, oder in Gegenwart eines säurebindenden Mittels mit einem Dichlornitroanilin der Formel

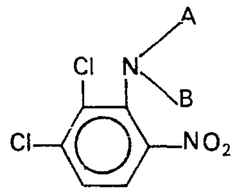

(III)

in einem inerten Lösungsmittel umsetzt, oder dass man

b) zur Herstellung solcher Verbindungen, in denen A einen chlorsubstituierten Alkylrest bedeutet, in einer entsprechenden Hydroxyalkylverbindung die Hydroxygruppe nach üblichen Methoden durch Chlor ersetzt, oder dass man

c) zur Herstellung solcher Verbindungen, in denen A die Trifluoracetylgruppe bzw. A und B gemeinsam die Gruppe $=CH\text{-}N(CH_3)_2$ bedeuten, die entsprechende substituierbare Verbindung der Formel I mit Trifluoracetanhydrid bzw. einem Dimethylformamiddialkylacetal oder Dimethylformamid/Phosphoroxychlorid umsetzt.

7. Herbizide Mittel, dadurch gekennzeichnet, dass sie neben einem Wirkstoff nach Anspruch 1 oder 2 ein oder mehrere bekannte herbizide Wirkstoffe enthalten.

8. Verwendung von Verbindungen nach Anspruch 1 oder 2 in Kombination mit einem oder mehreren bekannten Herbiziden.

**Claims:**

1. 2-Chloro-6-nitroanilines of the formula:

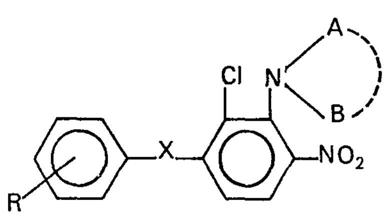

(I),

wherein A represents hydrogen, a straight-chained or branched alkyl group with 1 to 6 carbon atoms, a cycloalkyl group with 3 to 6 carbon atoms, a chlorine - or hydroxysubstituted alkyl group with 2 to 6 carbon atoms, allyl or trifluoroacetyl, B represents hydrogen, methyl, ethyl, n-pro pyl or isopropyl, A and B together also represent an alkylene with up to 5 members, possibly interrupted by oxygen, $=NH$ or $=NCH_3$, or they represent the group $=CH\text{-}N(CH_3)_2$, R represents hydrogen, fluorine, chlorine, bromine, methyl or methoxy, and X represents oxygen or sulphur.

2. 2-Chloro-3-phenoxy-6-nitroaniline.

3. Herbicidal agents, characterised in that they contain a compound as claimed in claim 1 or 2.

4. Use of compounds as claimed in claim 1 or 2, for combating undesirable plant growth.

5. Use of compounds as claimed in claim 1 or 2, for selective control of weeds in maize, wheat, barley, rice, rape and potatoes.

6. Process for preparing compounds as claimed in claim 1 or 2, characterised in that

a) a phenol or thiophenol of formula

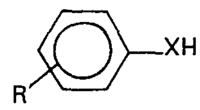

(II),

possibly in the form of a salt, more particularly an alkali salt, or in the presence of an acid-binding agent, is reacted, in a manner known per se, in an inert solvent, with a dichloronitroaniline of formula

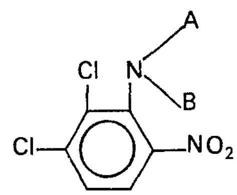

(III)

or b) in order to prepare compounds wherein A represents a chlorine-substituted alkyl group, in a corresponding hydroxyalkyl compound the hydroxy group is replaced by chlorine according to conventional methods,

or c) in order to prepare compounds wherein A represents the trifluoroacetyl group or A and B together represent the group $=CH\text{-}N(CH_3)_2$, the corresponding substitutable compound of formula I is reacted with trifluoroacetanhydride or a dimethylformamide dialkylacetal or dimethylformamide/phosphorus oxychloride.

7. Herbicidal agents, characterised in that they contain one or more known herbicidal agents in addition to an active substance as claimed in claim 1 or 2.

8. Use of compounds as claimed in claim 1 or 2, in conjunction with one or more known herbicides.

**Revendications**

1. 2-chloro-6-nitroanilines de formule

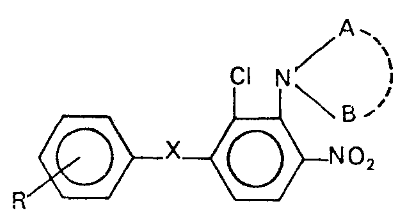

(I),

dans laquelle A représente l'hydrogène, un radical alcoyle rectiligne ou ramifié avec 1 à 6 atomes de C, un radical cycloalcoyle avec 3 à 6 atomes de C, un radical alcoyle chloro- ou hydroxysubstitué avec 2 à 6 atomes de C, allyle ou trifluoroacétyle, B représente l'hydrogène, méthyle, éthyle, n-propyle ou isopropyle, A et B pouvant également représenter ensemble un radical alcoylène éventuellement interrompu par l'oxygène, $=NH$ ou $=NCH_3$ avec jusqu'à 5 membres ou le groupe $=CH\text{-}N(CH_3)_2$,

R représente l'hydrogène, le fluor, le chlore, le brome, méthyle ou méthoxy et

X représente l'oxygène ou le soufre.

2. La 2-chloro-3-phénoxy-6-nitroaniline.

3. Agent herbicide, caractérisé par une teneur en un composé selon la revendication 1 ou 2.

4. Utilisation de composés selon la revendication 1 ou 2 pour combattre la végétation indésirable.

5. Utilisation de composés selon la revendication 1 ou 2 pour combattre de façon sélective les mauvaises herbes et les graminées indésirables dans le maïs, le froment, l'orge, le riz, les raves et les pommes de terre.

6. Procédé pour la préparation des composés selon la revendication 1 ou 2, caractérisé en ce que

a) on fait réagir, de manière en soi connue, un phénol ou thiophénol de formule

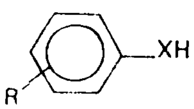

(II),

éventuellement sous forme d'un sel, en particulier un sel alcalin, ou en présence d'un agent fixant les acides avec une dichloronitroaniline de formule

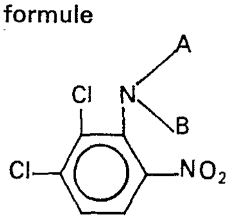

(III)

dans un solvant inerte, ou en ce que

b) pour la préparation de composés dans lesquels A représente un radical alcoyle chlorosubstitué, on remplace par du chlore, selon des méthodes usuelles, le groupe hydroxy dans un composé hydroxyalcoylé correspondant ou en ce que

c) pour la préparation de composés dans lesquels A représente le groupe trifluoroacétyle ou respectivement A et B représentent ensemble le groupe $=CH-N(CH_3)_2$, on fait réagir le composé de formule I substituable correspondant avec l'anhydride trifluoroacétique ou respectivement un dialcoylacétal du diméthylformamide ou le diméthylformamide/oxychlorure de phosphore.

7. Agents herbicides, caractérisés en ce qu'ils contiennent, outre une substance active selon la revendication 1 ou 2, une ou plusieurs substances actives herbicides connues.

8. Utilisation de composés selon la revendication 1 ou 2 en combinaison avec un ou plusieurs herbicides connus.